# EUROPEAN PATENT APPLICATION

(11) **EP 2 481 817 A1**
(43) Date of publication of application: **01.08.2012**
(21) Application number: 12165418.0
(22) Date of filing: 27.05.2008
(51) Int. Cl.: C12Q 1/68

(54) **Process for detecting nucleic acids**

(30) Priority: 15.06.2007 DE 102007027654
(62) Divisional of application: 08760101.9
(71) Applicant: GNA Biosolutions GmbH, 80799 München (DE)
(72) Inventor: Stehr, Joachim A., 80799 Muenchen (DE); Heindl, Dieter, 82396 Paehl (DE); Nichtl, Alfons, 82383 Hohenpeissenberg (DE); Klar, Thomas, A., 4020 Linz (AT); Feldmann, Jochen, 85232 Bergkirchen (DE); Hrelescu, Calin, 4040 Linz (AT); Parak, Wolfgang, 35037 Marburg (DE); Raschke, Gunnar, 80939 Muenchen (DE); Sperling, Ralf, 65343 Eltville (DE); Wunderlich, Michael, 82377 Pensberg (DE); Kuerzinger, Konrad, 83277 Penzberg (DE)
(74) Representative: Huebner, Stefan Rolf

(57) **Abstract**

A process for detecting nucleic acids, having the following steps: providing at least one nanoparticle that is functionalised for the nucleic acid to be detected by means of at least one oligonucleotide that is bound to it and that is able to hybridize with at least one segment of a nucleic acid to be detected; bringing the functionalised nanoparticle into contact with a sample in which the nucleic acid is to be detected; and measuring a property that provides information about the degree of hybridization of the at least one oligonucleotide with the nucleic acid to be detected. In addition, the process includes the step of exciting the nanoparticles to generate heat, for example by means of a photothermal effect.

The invention is suitable, in particular, for high-throughput DNA analysis.

## Description

### Background of the invention

The present invention relates to a process for detecting nucleic acids, according to the precharacterising portion of Claim 1. In addition, it relates to a kit for detecting nucleic acids, according to the precharacterising portion of Claim 20.

### State of the art

Many processes for detecting nucleic acids are based on the technique of melting-curve analysis. With this technique the effect is exploited that double-stranded nucleic-acid chains are able to dehybridize into single-stranded chains in the event of an increase in temperature, a process which in this context is described as 'melting'. The melting temperature depends, inter alia, on the degree of complementarity of the two hybridization partners.

From US published application US 2004/0219520 A1 and from the paper by C. Mirkin et al. entitled 'One-Pot Colorimetric Differentiation of Polynucleotides with Single Base Imperfections Using Gold Nanoparticle Probes', J. Am. Chem. Soc., 1998, Vol. 120, pages 1959-1964, a process for detecting nucleic acids is known in which use is made of gold nanoparticles that are functionalised with oligonucleotides. The nucleic acids to be detected and the functionalised gold nanoparticles are dissolved or suspended in an aqueous sample medium. One fraction of the oligonucleotides has a base sequence that is able to hybridize with a first segment of the nucleic-acid molecules to be detected, and another fraction of the oligonucleotides has a base sequence that is able to hybridize with a second segment of the nucleic-acid molecules to be detected. By reason of the hybridization, the nanoparticles of the nucleic acids are connected so as to form large aggregates, resulting in a broadening and red shift of their particle plasmon resonance. The latter is capable of being determined by light-extinction measurements. Now if the temperature of the sample is increased stepwise, a dehybridization - and, as a consequence, a dissolution of the aggregates - occurs at a melting temperature that is characteristic of the nucleic acid to be detected. With a melting curve that indicates the light extinction as a function of the temperature, this can be observed as a steep transition. Mirkin et al. report that they were able to distinguish melting curves of nucleic acids, the segments of which were fully complementary to the base sequences of the oligonucleotides of the functionalised nanoparticles, from those nucleic acids which differed in one base. It is also reported to have been possible to detect fully complementary nucleic acids in a mixture of fully complementary nuclei acids differing in one base.

In the known process it can be disadvantageous that the determination of the melting curve takes up a considerable time, typically 30 minutes to 120 minutes, because after each temperature step a wait has to be observed until a uniform temperature and an equilibrium between hybridized and dehybridized nucleic acids have arisen in the sample. In addition, the known process can cause difficulties in detecting nucleic acids that differ in one base in a mixture of fully complementary nucleic acids differing in one base. Moreover, from the paper by A. O. Govorov et al. entitled 'Generating heat with metal nanoparticles', nanotoday, 2007, Vol. 2, No. 1, pages 30-38, it is known that gold nanoparticles and silver nanoparticles can be excited to generate heat by being illuminated with light. In this paper it is also reported that in the case of identical excitation the heat generated by two adjacent gold particles is greater than the heat generated by two individual particles. This cooperative effect is ascribed to a Coulomb interaction between the adjacent nanoparticles.

In the paper by J. L. West et al. entitled 'Nanoshell-mediated near-infrared thermal therapy of tumors under magnetic resonance guidance', PNAS, 2003, Vol. 100, No. 23, pages 13549-13554, nanoparticles consisting of silica particles surrounded by a gold shell ('nanoshells') are known that generate heat, particularly under illumination with infrared light. The authors propose to employ the nanoparticles for the thermal dissolution of tumours.

Lastly, Jacobson et al. disclose in 'Remote electronic control of DNA hybridization through inductive coupling to an attached metal nanocrystal antenna', Nature, 2002, Vol. 415, pages 152-155, a gold nanoparticle that is covalently bonded to a loop-shaped nucleic-acid segment which connects the self-complementary ends of a hairpin-shaped DNA molecule to one another. The gold nanoparticle is excited to generate heat by means of inductive coupling to a magnetic radio-frequency field, in order to increase the local temperature of the DNA molecule that is bound to the nanoparticle and in this way to induce a dehybridizing of the self-complementary ends. In the same paper, Jacobson et al. also disclose a process in which oligonucleotides are bound to a gold nanoparticle at their one end and bear a fluorophore at their other end. Oligonucleotides complementary thereto are bound to a streptavidin-jacketed agarose bead. The two complementary oligonucleotides hybridize. Subsequently a dehybridization is induced, specifically either by local increase of temperature by means of exciting the gold nanoparticles in a magnetic radio-frequency field or by increasing the temperature of the sample. In each case the degree of hybridization is ascertained on the basis of the fluorescence of the supernatant.

### Problem underlying the invention

The object underlying the invention is to provide an improved process for detecting nucleic acids. The object further underlying the invention is to provide an improved kit for detecting nucleic acids.

### Solution according to the invention

For the purpose of achieving the object, the invention teaches a process for detecting nucleic acids, said process having the features of Claim 1. In addition, the invention teaches a kit for detecting nucleic acids, said kit having the features of Claim 20.

Nanoparticles in the sense of the present invention are particles that, by reason of their size, exhibit special optical properties, in particular characteristic absorption spectra or scattering spectra which do not appear - or do not appear so clearly - in bulk material. The metal nanoparticles disclosed by A. O. Govorov et al., referenced above, and those disclosed by J. M. Jacobson et al., referenced above, are named merely in exemplary manner. The content of the aforementioned documents in this regard is part of the present disclosure by reference. The nanoparticles have a diameter of less than 500 nm, preferably less than 100 nm. Particularly preferred nanoparticles have a diameter between 5 nm and 80 nm.

The nanoparticles may be globular, but non-globular shapes, in particular, also enter into consideration, for example rod-like nanoparticles. Processes for producing and functionalising the nanoparticles are known, for example, from the paper by Mirkin et al., referenced above, and from the further references stated therein, the content of which in this regard is part of the present disclosure by reference.

The term 'oligonucleotide' in connection with the present invention preferably encompasses not only deoxyoligoribonucleotides but also oligonucleotides that contain one or more nucleotide analogues with modifications on their backbone (e.g. methylphosphonates, phosphothioates or peptide nucleic acids [PNA]), in particular on a sugar of the backbone (e.g. 2'-O-alkyl derivatives, 3'- and/or 5-aminoriboses, locked nucleic acids [LNA], hexitol nucleic acids or tricyclo-DNA; in this connection, see the paper by D. Renneberg and C. J. Leumann entitled 'Watson-Crick base-pairing properties of tricyclo-DNA', J. Am. Chem. Soc., 2002, Vol. 124, pages 5993-6002, the content of which in this regard is part of the present disclosure by reference), or contain the base analogues, for example 7-deazapurine or universal bases such as nitroindole or modified natural bases such as N4-ethylcytosine. In one embodiment of the invention, the oligonucleotides are conjugates or chimeras with non-nucleosidic analogues, for example PNA. In one embodiment of the invention, the oligonucleotides contain, at one or more positions, non-nucleosidic units such as spacers, for example hexaethylene glycol or Cₙ spacers, where n is between 3 and 6. To the extent that the oligonucleotides contain modifications, these are chosen in such a way that a hybridization with natural DNA/RNA analytes is possible also with the modification. Preferred modifications influence the melting behaviour, preferably the melting temperature, in particular in order to be able to distinguish hybrids having differing degrees of complementarity of their amino acids (mismatch discrimination). Preferred modifications encompass LNA, 8-aza-7-deazapurine, 5-propinyluracil, 5-propinylcytosine and/or non-basic interruptions in the oligonucleotide.

In the sense of the present invention, the term 'hybridizing' means the formation of a double strand. With the invention it is possible to ensure that the double strands dehybridize ('melt') at least partly as a result of a local increase in temperature, for example by reason of the exciting of the nanoparticles to generate heat. In one embodiment of the invention, the oligonucleotide is chosen in such a way that it dehybridizes from the nucleic acid to be detected at a melting temperature below 80 °C, preferably distinctly below 80 °C. The oligonucleotide is preferably chosen in such a way that it dehybridizes from the nucleic acid to be detected at a melting temperature greater than 40 °C. The melting temperature is the temperature at which the melting curve exhibits the maximum magnitude of the gradient (extreme point of the derivative of the melting curve). Preferred oligonucleotides are, in addition, chosen in such a way that they are sufficiently specific to the nucleic acid in order to detect it. A person skilled in the art can adjust the melting-point and the specificity, inter alia, on the basis of the length of the nucleotide. Preferred oligonucleotides have a length between 8 bases and 40 bases, particularly preferably between 12 bases and 25 bases. The preferred oligonucleotide is at least partly complementary, particularly preferably totally complementary, or complementary with the exception of one or two bases, to a segment of the nucleic acid to be detected.

It is an attainable advantage of the invention that as a result of the exciting of the nanoparticles to generate heat a local heating of the sample can be obtained in the vicinity of the nanoparticles. In this way, a melting can be triggered without the entire sample having to be heated for this purpose. By virtue of the local heating, the hybridization region within the aggregates of nanoparticles, discussed further below, can be heated up very quickly, for example within a few µs, from an initial temperature to a desired local temperature, in order to check whether this results in melting. It is an attainable advantage of the invention that a melting curve can be recorded more quickly than in the state of the art.

The invention is suitable, in particular, for application in multiwell processes and in high-throughput DNA analysis.

### Structure and further development of the solution according to the invention

Advantageous designs and further developments that can be employed individually or in combination are the subject-matter of the dependent claims.

In a preferred embodiment of the invention, the nanoparticle is excited to generate heat with electromagnetic radiation, for example with light, preferably by means of an optothermal effect. Preferred light-sources are lasers, light-emitting diodes (LEDs) and flash lamps. The light-source may emit the light in pulsed manner or continuously. Both monochromatic and polychromatic light-sources, in particular white light-sources, enter into consideration. The term 'light' in the sense of the present invention includes the spectrum of electromagnetic radiation from the far infrared to the far ultraviolet. It is also conceivable to excite the nanoparticles with radio-frequency fields, for example with a magnetic radio-frequency field, preferably as described in Jacobson et al., referenced above.

In a preferred embodiment of the invention, the nanoparticle includes at least one metal, preferably a noble metal, for example gold or silver. In one embodiment the nanoparticle consists totally of the metal, in another the metal forms only a part of the nanoparticle, for example its sheath. An example of the latter embodiment is constituted by the silica/gold nanoshells disclosed by J. L. West, referenced above. The entire content of the aforementioned document in this regard is part of the present disclosure by reference. The nanoparticle is brought into contact with the nucleic acid to be detected, preferably by diffusion, particularly preferably in an aqueous medium in which the nucleic acid and the nanoparticle are dissolved or suspended. Preferably a plurality of nucleic acids and nanoparticles of the same type are dissolved or suspended in the medium, and the property provides information about the degree of hybridization of the plurality of oligonucleotides with the plurality of nucleic acids. The nanoparticles are preferably present in the medium in a concentration between 0.5 nM (nmol/litre) and 50 nM. The nucleic acids to be detected are preferably present in the medium in a concentration between 100 pM and 10 mM. In addition, a preferred medium contains a salt, preferably common salt (NaCl). The preferred salt concentration lies between 0.01 M and 1 M.

In one embodiment of the invention, at least some of the nanoparticles or some of the nucleic acids are immobilised on a substrate. It is an attainable advantage of this embodiment of the invention that negative influences of a thermal convection or of gravitative sedimentation effects in the sample on the results of measurement can be avoided.

The property that provides information about the degree of hybridization of the nucleic acid with the oligonucleotide is preferably an optical property, for example the colour or colour intensity of a colour marker, for example of a dyestuff molecule or of a colloidal semiconductor nanocrystal (quantum dot). For instance, a colour marker may be bound to one of the hybridization partners, preferably to the nucleic acid to be detected, or use may be made of a colour marker that is capable of being intercalated between the hybridization partners in the course of hybridization. In particularly preferred manner, with this embodiment of the invention the fact is exploited that certain fluorescence markers, in particular fluorescent dyes, in the vicinity of nanoparticles lose their fluorescence at least partially (quenching). With this embodiment of the invention, it is possible to ensure that the degree of hybridization can be inferred on the basis of the colour intensity or colour.

In a preferred embodiment of the invention, the property that provides information about the degree of hybridization is a property of the nanoparticle, preferably an optical property of the nanoparticle. The invention preferably exploits the fact that nanoparticles, by virtue of the fact that they are adjacent to one another, are able to change their optical properties, in particular in such a way that their extinction spectrum is spectrally shifted and/or widened.

In a preferred embodiment variant, the nucleic acid to be detected includes at least two segments, and at least two nanoparticles are provided, at least one of the nanoparticles being functionalised with an oligonucleotide that is able to hybridize with the first segment of the nucleic acid, and at least one of the nanoparticles being functionalised with an oligonucleotide that is able to hybridize with the second segment of the nucleic acid. The functionalised nanoparticles are brought into contact with a sample in which the nucleic acid is to be detected, and the property that provides information about the degree of hybridization of the oligonucleotides with the nucleic acid to be detected is measured. In this embodiment of the invention, it is possible to ensure that when the nanoparticles are connected to one another by the hybridization the relative closeness of the nanoparticles brings about a measurable change in their optical properties. The oligonucleotide-functionalised nanoparticles are preferably formed in such a way that they hybridize with the nucleic acid to be detected in a head-to-head configuration - i.e. the segments of the oligonucleotides with which they are bound to their nanoparticles are closest to one another in the hybridized state. But head-to-tail and tail-to-tail configurations are also conceivable, as disclosed, for example, in schema 1 of the paper by C. A. Mirkin, referenced above.

In another conceivable embodiment of the invention, both the at least one oligonucleotide and the nucleic acid to be detected are bound to a nanoparticle. Also with this embodiment of the invention it is possible to ensure that two nanoparticles are bound to another by virtue of the hybridization, which may result in a measurable change in the optical properties.

In the case of at least some of the nanoparticles, several oligonucleotides, in each instance, are preferably bound to a common nanoparticle. It is an attainable advantage of this embodiment of the invention that clusters consisting of three or more nanoparticles may arise in the course of the hybridization of the oligonucleotides with the nucleic acid. As a result, the fact that the change in the property that provides information about the degree of hybridization increases with the size of the aggregate can be exploited advantageously. In particular, this may alleviate the detection of the change in the property, and hence of the hybridization.

In a preferred process according to the invention, at least the following three steps are run through: a) measuring the property that provides information about the degree of hybridization of the nucleic acid with the oligonucleotides, at a predetermined initial temperature, b) exciting the at least one nanoparticle to generate heat; and c) renewed measuring of the property that provides information about the degree of hybridization of the nucleic acid with the oligonucleotide. With this embodiment of the invention, ascertaining a melting signal that is a measure of the change in the degree of hybridization by reason of the local heating of the sample can be ensured by comparison of the results of measurement before and after the exciting of the nanoparticles to generate heat.

In one embodiment of the invention, steps a) to c) are run through several times, whereby in the course of the passes the at least one nanoparticle is excited to generate heat in variably intense manner, preferably increasing from pass to pass, for example by illuminating with differing quantities of light. The melting signals ascertained in the course of the passes are preferably compared with one another. As a result, a melting-signal curve can be recorded that indicates the melting signal as a function of the degree of excitation of the nanoparticles. Preferably between 5 and 50 melting signals, particularly preferably between 10 and 20 melting signals, are recorded. From the melting-signal curve a melting threshold can be ascertained that indicates the degree of excitation at which melting begins. With this embodiment of the invention, detecting the nucleic acid on the basis of a melting threshold that is specific to the nucleic acid at given initial temperature and for given oligonucleotides can be ensured.

In a preferred process according to the invention, the melting threshold is determined for several initial temperatures, in order to ascertain a melting-threshold curve. To this end, steps a) to c) are preferably run through several times at a first predetermined initial temperature, whereby in the course of the passes the at least one nanoparticle is excited to generate heat in variably intense manner, and the melting signals of the passes are compared, in order to ascertain a first melting threshold. In addition, steps a) to c) are run through several times at a second predetermined initial temperature, whereby in the course of the passes the nanoparticle is excited to generate heat in variably intense manner, in order to ascertain a second melting threshold. In particularly preferred manner, the procedure is repeated at other initial temperatures, whereby for this purpose in particularly preferred manner the initial temperature is increased stepwise. With this embodiment of the invention, detecting the nucleic acid on the basis of a melting-threshold curve that is specific to the nucleic acid can be ensured.

The inventors have established that the melting threshold decreases monotonically with increasing initial temperature, at least within an initial-temperature range. They attribute this to the fact that with increasing initial temperature the additional local heating by virtue of the exciting of the nanoparticles, which is necessary in order to trigger melting, decreases. In one embodiment of the invention, the gradient of the melting-threshold curve is ascertained within a predetermined initial-temperature range. By this means, a detection of the nucleic acid on the basis of a gradient that is specific to the nucleic acid can be ensured. In another embodiment of the invention, the melting-threshold curve is linearly extrapolated to a zero point of the melting threshold. With this embodiment of the invention, it can be ensured that a nucleic acid is detected on the basis of a zero point that is specific thereto.

In the case of the nanoparticle aggregates that are formed by the hybridization of the nucleic acid to be detected with the oligonucleotides, at certain temperatures an annealing (aggregate growth) can occur, in the course of which the size of the aggregates increases. Particulars relating to this effect are disclosed in the paper by J. J. Storhoff et al. entitled 'What controls the optical properties of DNA-linked gold nanoparticle assemblies?', J. Am. Chem. Soc., 2000, Vol. 122, pages 4640-4650, the content of which in this regard is part of the present disclosure by reference. This annealing temperature may be specific to certain nucleic acids for given oligonucleotides. In one embodiment of the invention, the nucleic acid is therefore detected on the basis of an annealing temperature that is specific to the nucleic acid for given oligonucleotides.

The melting threshold may be a function of the aggregate size, for example because an aggregate is unable to emit the heat to the environment as quickly as a single nanoparticle, resulting in a build-up of heat in the aggregate. A cooperative effect of several nanoparticles also enters into consideration as a cause, which has the result that aggregates of nanoparticles generate more heat, given the same excitation, than individual nanoparticles; see the paper by A. O. Govorov et al., referenced above, the content of which in this regard is part of the present disclosure by reference. The melting threshold preferably declines with the size of the aggregate. In one embodiment of the invention, the nucleic acid is therefore detected by virtue of the fact that a melting threshold at an initial temperature that is substantially greater than or equal to the annealing temperature lies below a certain value. Alternatively, the nucleic acid can be detected by virtue of the fact that below the annealing temperature a melting threshold after an annealing process is lower than before it (hysteresis). For example, at least one melting threshold can be ascertained at at least one initial temperature below the annealing temperature, then the initial temperature can be raised temporarily to or above the annealing temperature, and then once again at least one melting threshold can be ascertained at at least one initial temperature below the annealing temperature. By the comparison of the melting thresholds, an annealing, and hence the nucleic acid to which the annealing temperature is specific, can be detected.

With the invention it is also possible to detect several different nucleic acids in the same sample. If, for example, the first nucleic acid has a melting temperature that lies below the melting temperature of the second nucleic acid to be detected, the first nucleic acid can be detected by the presence of a melting threshold at a temperature below its melting temperature, and the second nucleic acid can be detected by detection of a melting threshold at a temperature below the melting temperature of the second nucleic acid but above the melting temperature of the first nucleic acid. In one embodiment of the invention, steps a) to c) are therefore run through at least one first and one second time, the predetermined initial temperature being variable in the course of the passes, and the melting signals of the passes being compared.

In order to be able to distinguish the first nucleic acid from the second nucleic acid at the first initial temperature on the basis of its melting threshold, it may be advantageous to exploit the fact that the first nucleic acid displays an annealing at or even already below the first temperature, but the second nucleic acid does not. This is because, as explained above, the annealing can result in a distinct lowering of the melting threshold. In a particularly preferred embodiment of the invention, the first temperature therefore lies at or above an annealing temperature of the first nucleic acid to be detected.

It is also conceivable to detect several differing nucleic acids through the use of differing nanoparticles that have differing excitation properties, for example inasmuch as they generate differing quantities of heat in the case of identical excitation. In a preferred embodiment of the invention, several fractions of nanoparticles are therefore provided that have differing excitation properties, for example inasmuch as they react to the same excitation with differing heating, and the nanoparticles of a first fraction are functionalised for a first nucleic acid to be detected, and the nanoparticles of the second fraction are functionalised for a second nucleic acid to be detected, which is different from the first nucleic acid. The nanoparticle fractions may, for example, differ by virtue of the fact that the nanoparticles have differing size or consist of differing materials or material combinations or have differing proportions of a certain material or of a certain material combination. By virtue of the fact that the nanoparticles display differing excitation properties, the fractions can be distinguished. If the various nanoparticles generate differing quantities of heat, for example in the case of identical excitation, the melting thresholds are shifted relative to one another and are characteristic in each instance of the associated nanoparticle fraction. It is also conceivable that the nanoparticles of one fraction react more intensely to an excitation of one type, for example electromagnetic radiation of one wavelength, and the nanoparticles of another fraction more intensely to an excitation of another type, for example electromagnetic radiation of another wavelength. In one embodiment of the invention, the fractions are therefore excited by differing types of excitation. To this end, preferably two, three or more excitation sources are provided, particularly preferably lasers of differing wavelength. As a result, it can be ensured that the melting threshold for the first nucleic acid to be detected differs distinctly from that for the second nucleic acid to be detected. As a result, several nucleic acids can be detected in the same sample. Of course, third, fourth or further fractions with nanoparticles may also be provided, which again generate differing quantities of heat in the case of the same excitation. In this way, numerous differing nucleic acids can be detected.

### Brief description of the drawings

In the following, the invention will be elucidated in more detail in further particulars on the basis of schematic drawings in respect of exemplary embodiments.

Shown are:
- Fig. 1:: schematically, an embodiment of the invention in which the nanoparticles are excited to generate heat by being illuminated with laser light and in which information about the degree of hybridization in the sample is obtained by measuring a transmission of light through the sample;
- Fig. 2:: schematically, an embodiment of the invention in which the degree of hybridization is measured by measuring the fluorescence (photoluminescence) of a dye in the sample;
- Fig. 3:: schematically, an embodiment of the invention in which the degree of hybridization is inferred by measuring the intensity of scattered light;
- Fig. 4:: schematically, an aggregate of functionalised gold nanoparticles connected by means of nucleic acids to be detected;
- Fig. 5:: extinction curves of isolated gold nanoparticles and of gold nanoparticles connected so as to form aggregates;
- Fig. 6:: melting curves of gold-nanoparticle aggregates which are connected either to totally complementary nucleic acids or to nucleic acids that are totally complementary with the exception of one base;
- Fig. 7:: schematically, gold-nanoparticle aggregates which diffuse freely in a solution;
- Fig. 8:: schematically, gold-nanoparticle aggregates which are localised on a substrate;
- Fig. 9:: an example of the change in the extinction of a sample with gold-nanoparticle aggregates after the latter have been excited to generate heat by being illuminated with light;
- Fig. 10:: an example of the change in the extinction after the nanoparticle aggregates have been illuminated repeatedly;
- Fig. 11:: an example of a melting-signal curve with a melting threshold;
- Fig. 12:: an example of a melting-threshold curve without hysteresis;
- Fig. 13:: an example of a melting-threshold curve with hysteresis;
- Fig. 14:: the comparison of two melting-threshold curves having the same gradient, in which the zero point of the melting threshold has been determined by extrapolation;
- Fig. 15:: an example of the determination of two different nucleic acids in the same sample by measuring two melting signals at differing initial temperatures;
- Fig. 16:: an example of the determination of two different nucleic acids by means of two nanoparticle fractions that differ in the size of the nanoparticles.

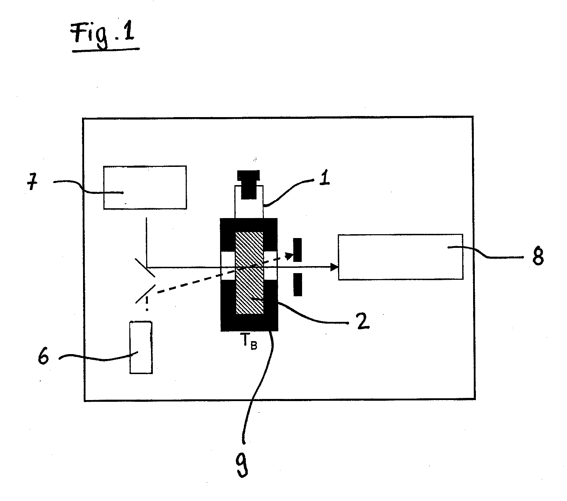

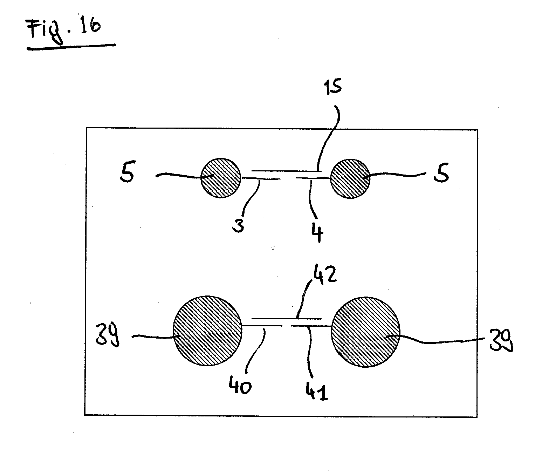

In Fig. 1 an example of the invention can be seen in which there is contained in a cell 1 by way of sample container a sample in which nanoparticles 5 functionalised with oligonucleotides 3, 4 (see Fig. 16) are suspended and are able to diffuse freely. More precisely, the sample contains 6 nM gold nanoparticles 5 which have been functionalised with a first oligonucleotide 3 that is able to hybridize with a first segment of the nucleic acid to be detected, and 6 nM gold particles which have been functionalised with a second oligonucleotide 4 that is able to hybridize with a second segment of the nucleic acid to be detected. The sample further contains 240 nM of the nucleic-acid molecule to be detected and 300 nM NaCl. The gold nanoparticles 5 can be excited to generate heat with a pulsed Nd:YLF laser-light source 6 by means of pulses, 300 ns in length, having a wavelength of 527 nm. A diode laser 7 having a wavelength of 650 nm, which transilluminates the sample 2, and the light of which subsequently falls onto a fast photodiode 8, serves for measuring an extinction of the sample 2 at this wavelength.

In addition, the sample cell 1 stands in a water bath 9 with which the initial temperature T_{B} of the sample 2 can be adjusted.

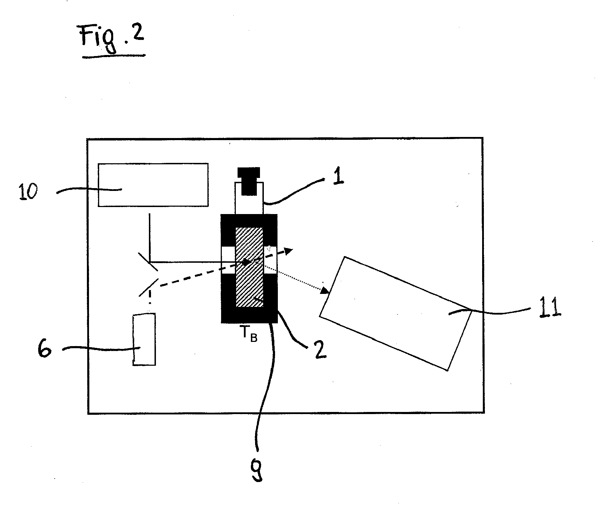

Fig. 2 shows a modification of the example from Fig. 1, in which for the purpose of detecting the hybridization the fluorescence of a fluorescent dye in the sample 2 is measured, rather than the extinction or transmission. Provided to this end are a light-source 10, which is able to excite the fluorescent dyes in the sample 2, and a photoluminescence detector 11 for measuring the intensity of the fluorescent light.

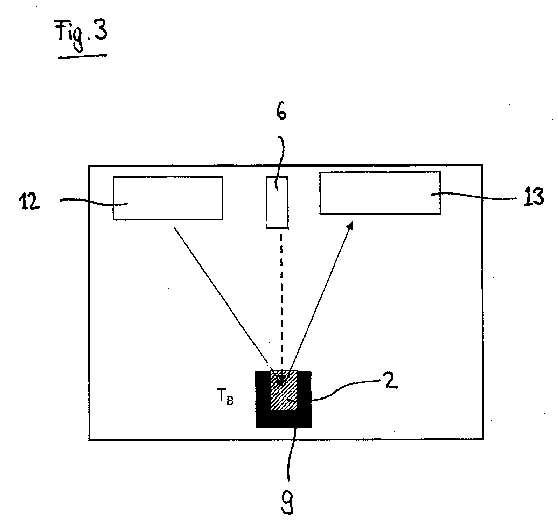

In Fig. 3 a further exemplary embodiment can be seen, in which the intensity of the light scattered by the sample 2 is measured, instead of the extinction of light or the fluorescence. Provided for this purpose are a scanning light-source 12, which beams light into the sample, and a scattered-light detector 13, which measures the intensity of light scattered out of the sample 2. An advantage of this embodiment is the fact that no sample container is required that is transparent to the light on two sides.

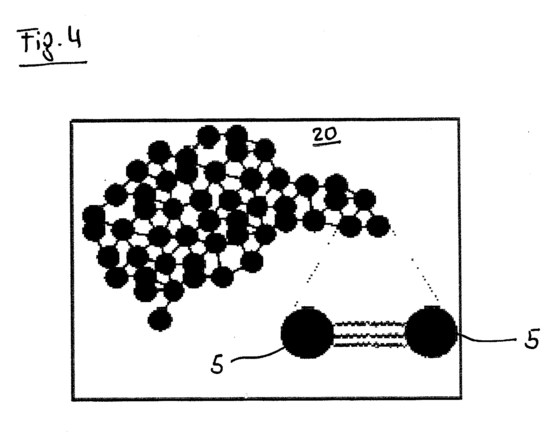

The way in which nanoparticles 5 - functionalised with, in each instance, several oligonucleotides 3, 4 - of nucleic acids 15 to be detected can be connected so as to form aggregates 20 is reproduced schematically in Fig 4. In the enlarged example, at bottom right in the Figure, two nanoparticles 5 are connected by means of three hybrids in a tail-to-tail configuration. To this end, the nucleic acid 15 exhibits two segments, one of which is at least partly complementary to an oligonucleotide 3 attached to a first gold particle 5, and another of which is at least partly complementary to an oligonucleotide 4 attached to a second gold particle 5. The spacing between the nanoparticles amounts to about 20 nm.

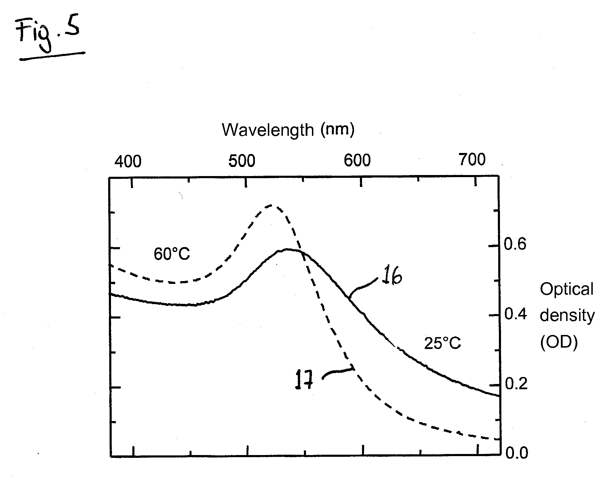

Fig. 5 shows the result of a measurement of the optical density (OD) as a measure of the extinction of the gold nanoparticles 5 in the sample 2 at two different initial temperatures, 25 °C 16 and 60 °C 17, which were adjusted by the water bath 9. The gold nanoparticles have a diameter of 10 nm and have been functionalised with thiol oligonucleotides having a length of 30 base pairs. It can be discerned that in the case of an increase in temperature the extinction spectrum shifts towards shorter wavelengths and becomes narrower. In the literature this is attributed to an at least partial dissolution of the gold-nanoparticle aggregates 20 and to an associated change in the particle plasmon resonance of the gold nanoparticles 5. The dissolution is, in turn, a consequence of the melting of the hybrids.

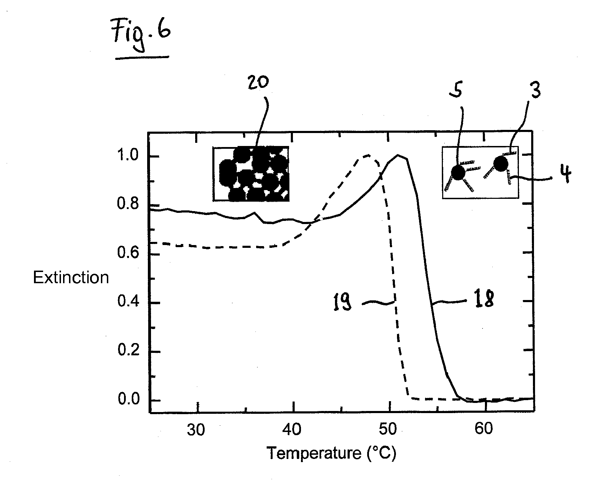

From the extinction it is possible, as represented more precisely in Fig. 6, to infer the melting temperature. Fig. 6 shows the normalised extinction at a wavelength of 650 nm of the light-source 7 as a function of the initial temperature, adjusted by the water bath 9, for two different nucleic acids 15, the gold nanoparticles 5 having been functionalised with the same oligonucleotides 3, 4. The nucleic acids 15 differ by virtue of the fact that the nucleic-acid segments of the first melting curve 18 are totally complementary to the oligonucleotides 3, 4 of the nanoparticles 5, whereas a nucleic-acid segment of the second melting curve 19 is not complementary at one base.

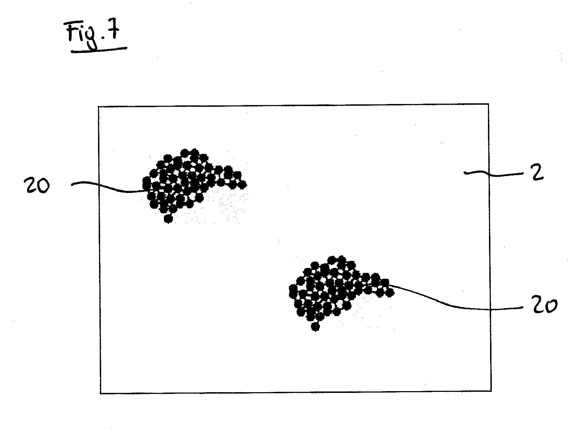

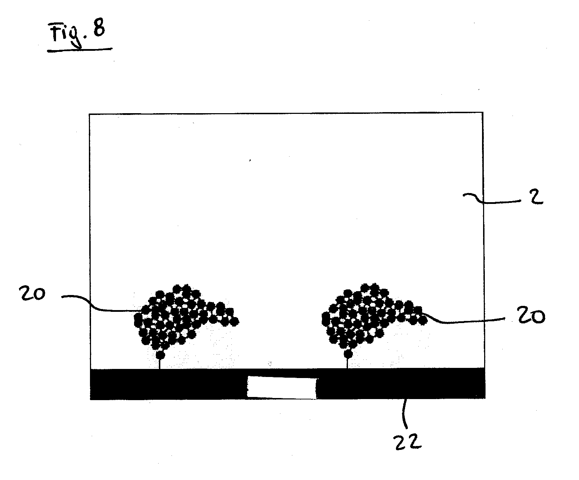

The curves 18, 19 show, for both nucleic acids 15, a steep decline in the extinction at the melting temperature. However, the nucleic acid 15 that is not complementary in one base has a distinctly lower melting temperature (about 50.5 °C) than the totally complementary nucleic acid (54 °C). In the examples described above, the aggregates 20 have been suspended in the sample 2 and are able to diffuse freely therein, as represented in Fig. 7. In another example, represented in Fig. 8, some of the functionalised gold nanoparticles 5 have been localised on a substrate 22. By this means, disadvantageous effects of thermal convection or gravitative sedimentation effects in the sample 2 can be avoided, for example.

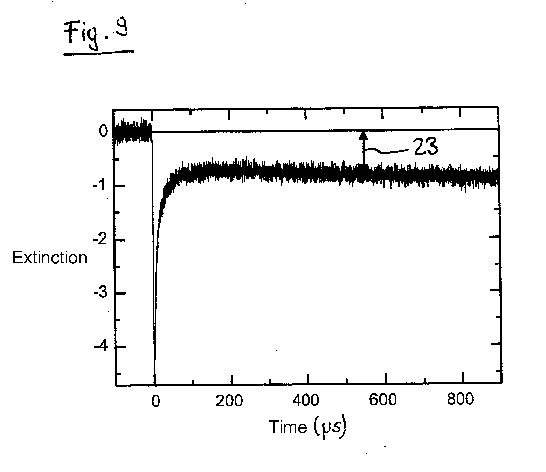

Fig. 9 shows, in relative units, the change in the extinction at a wavelength of 650 nm in the sample 2 from the example in Fig. 1 after said sample has been illuminated by the laser 6 with a laser pulse having a peak power of 3.8 kW/mm² and at an initial temperature, adjusted with the water bath 9, of 25 °C. The extinction was measured at a wavelength of 650 nm. At first, the extinction decreases considerably, this being appraised by the inventors as a consequence of a thermally induced broadening of the particle plasmon resonance of the gold nanoparticles 5. This signal decays with a time constant of 11 µs. There remains a long-persisting signal, which points to an at least partial dissolution of the aggregates 20, caused by melting. The difference in the extinction before and after the exciting of the nanoparticles 5 is the melting signal 23.

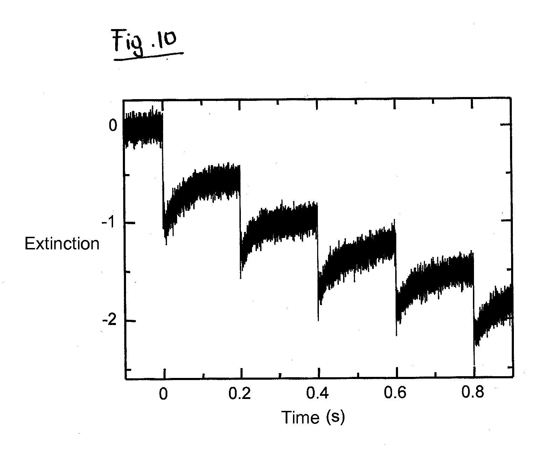

Fig. 10 shows, in relative units, the change in the extinction at a wavelength of 650 nm in the case of a string of five aggregates of the nanoparticles 5 at a repetition-rate of 5 Hz (note the zoom factor of 1000 in the time-scale in comparison with Fig. 9). The stepwise decrease in the extinction is the consequence of an accumulation of dissociated aggregates 20 in the sample 2. The decrease in the extinction that follows is presumably partly a result of a re-hybridization and partly a result of a diffusion of non-dissociated aggregates 20 out of the sample 2 into the region in which the sample 2 is transilluminated by the light-source 7.

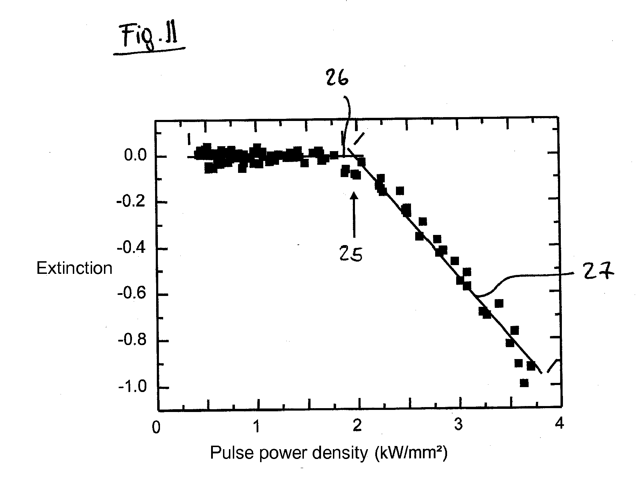

The melting-signal curve represented in Fig. 11 has arisen by virtue of the fact that a change in the extinction, measured in each instance 550 µs after the excitation of the nanoparticles 5, has been plotted in relative units against the pulse power density of the laser 6 that was employed. Below a threshold of about 2 kW/mm² pulse power density of the laser 6 no measurable melting signal 23 can be observed. After this, a substantially linear decrease in the melting signal as a function of the power can be discerned. The melting threshold 24 can be ascertained by determining a point of intersection 25 of the linear fits in the region without measurable signal 26 and in the substantially linearly declining region 27.

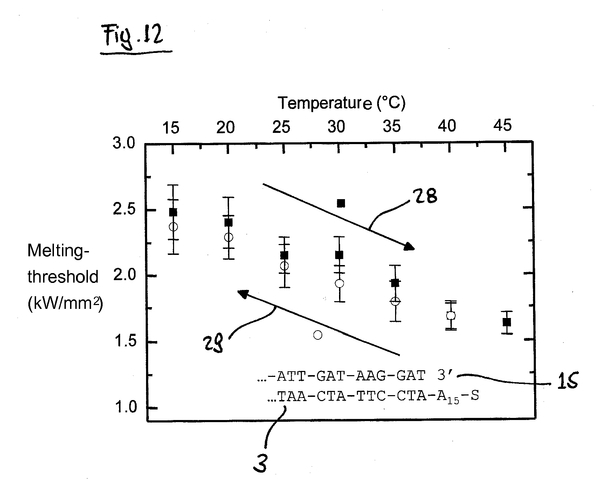

Fig. 12 shows the dependency of the melting threshold on the initial temperature which is adjusted by the water bath 9. The melting threshold decreases with increasing initial temperature. The closer the initial temperature is to the melting temperature, the smaller the increases in temperature that have to be induced by means of excited nanoparticles 5 in order to trigger melting. In order to exclude size-dependent effects on the melting threshold by reason of aggregate size growing with time, the melting thresholds were measured once with increasing temperature 28 and once with decreasing temperature 29. The totally complementary nucleic acid 15 was located in the sample 2 by way of nucleic acid to be detected. The bidirectional measurement showed identical results, within measuring errors.

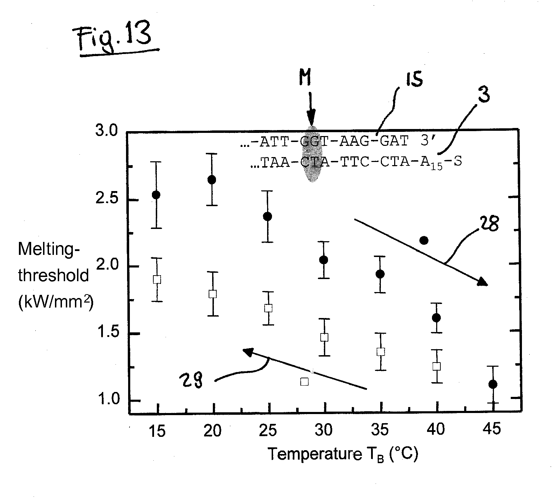

Fig. 13 shows the same measurement with a nucleic acid 15 that is not complementary with one base (M in the Figure). In this case, unlike in Fig. 12, the melting threshold declines greatly at a temperature of 45 °C, and a hysteresis becomes evident if the temperature is lowered again 29. The hysteresis is attributed to an annealing at an annealing temperature of 45 °C. The increase in the size of the aggregates 20, caused by the annealing, lowers the melting threshold, because the gold nanoparticles bring about a greater increase in temperature with identical excitation, inter alia on account of a decreasing surface/volume ratio of the aggregates. With the aid of the annealing effect, therefore, differing nucleic acids 15 can be distinguished from one another.

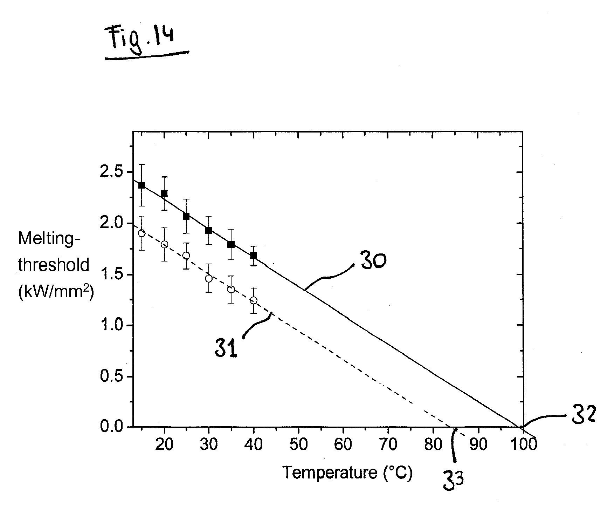

In Fig. 14, portions of the melting-threshold curves for the totally complementary nucleic acid 28 and for the nucleic acid differing in one base have been extrapolated 30, 31 after the annealing 29. The melting-threshold zero points 32, 33 ascertained by the extrapolation are different and can be drawn upon for the purpose of detection or for the purpose of distinguishing the nucleic acids 15.

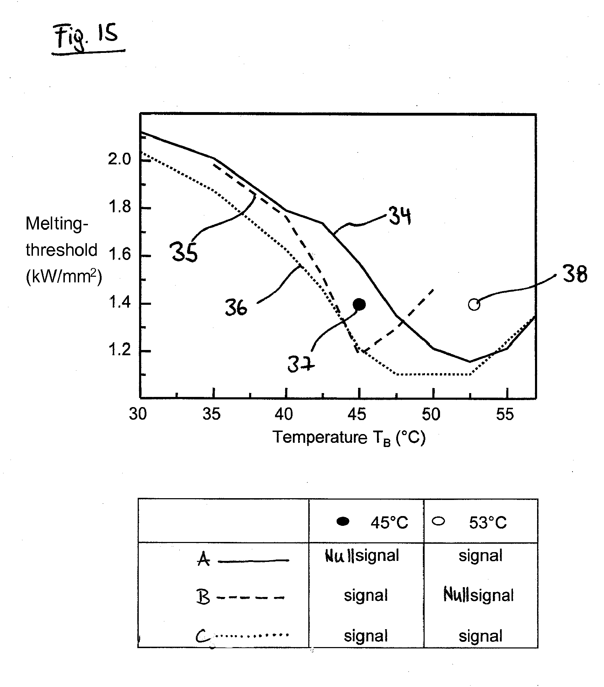

Fig. 15 clarifies the manner in which two nucleic acids 15 in the same sample 2 can be detected with only two excitations of the nanoparticles 5 at two different initial temperatures. Reproduced to this end are the melting-threshold curve 34 of a sample 2 that exclusively contains totally complementary nucleic-acid molecules, the melting-threshold curve 35 of a sample 2 that exclusively contains nucleic-acid molecules differing in one base, and the melting-threshold curve 36 of a sample that contains a 1:1 mixture of both nucleic acids.

Both the melting-threshold curve 35 and the melting-threshold curve 36 display a striking decline in the melting threshold at 45 °C, the annealing temperature of the nucleic acid 15 differing in one base. On the other hand, in the case of the mixture another melting threshold is detectable also at 53 °C, whereas this is not the case with the sample 2 that contains nucleic acids differing only in one base, because in this case all the aggregates are already dissociated. In this manner, with only two excitations at two different temperatures the three samples 2 can be distinguished in the following way: the two pairs constituted by excitation power and initial temperature have been represented in Fig. 15 as points 37, 38 (laser power density 1.4 kW/mm², temperature 45 °C and 53 °C, respectively). The results are presented in the table given underneath. In case A of the sample 2 that contains only totally complementary nucleic acids, at 45 °C no melting signal is measured that indicates a melting, because the power density of the excitation lies below the melting threshold. However, at 53 °C a melting is observed, since here the melting threshold is lower. In case B of the sample 2 that contains nucleic acids differing only in one base, the shift of the melting threshold at 45 °C is great enough to obtain a melting signal there already. At 53 °C, on the other hand, no melting signal is established any longer, because this temperature lies above the melting temperature and therefore all the aggregates 20 are already dissociated even without excitation. In case C of the mixed sample 2, both measurements result in a melting signal: at the low temperature by reason of the melting threshold - which is lowered by the annealing - of the nucleic acid differing in one base; and at the high temperature by reason of the as yet not totally dissociated aggregates of the totally complementary nucleic acid. The record consequently permits the three samples to be clearly distinguished, without time-consuming stepwise increases in the initial temperature being necessary. In principle, the method can be extended in appropriate manner to more than two different nucleic acids to be detected.

Fig. 16 clarifies the manner in which different nucleic acids can be distinguished through the use of nanoparticles 5, 39 of differing size. A first fraction of nanoparticles 5 is functionalised with oligonucleotides 3, 4 for a first nucleic acid 15. A second fraction of nanoparticles 39 is functionalised with other oligonucleotides 40, 41 for another nucleic acid 42. Because the different nanoparticles 5, 39 generate differing quantities of heat in the case of identical excitation, the melting threshold is clearly different in the two fractions. In principle, this process can also be extended by means of further fractions of nanoparticles to a greater number of nucleic acids to be detected. It is also conceivable to combine the process represented in exemplary manner in Fig. 16 with the process represented in exemplary manner in Fig. 15, in order to detect a still greater number of nucleic acids.

The features disclosed in the above description, in the Claims and in the drawings may be of significance, both individually and in arbitrary combination, for the realisation of the invention in its various configurations.

## Claims

1. A process for detecting nucleic acids, having the following steps:
- providing at least one nanoparticle that is functionalised by means of at least one oligonucleotide that is bound to it and that is able to hybridize with at least one segment of a nucleic acid to be detected,
- bringing the functionalised nanoparticle into contact with a sample in which the nucleic acid is to be detected, and
- measuring a property that provides information about the degree of hybridization of the at least one oligonucleotide with the nucleic acid to be detected,
**characterised in that**
in addition the process includes the step of exciting the nanoparticles to generate heat.

2. Process according to Claim 1,
**characterised in that**
electromagnetic radiation is applied to excite the nanoparticle to generate heat.

3. Process according to Claim 1 or 2,
**characterised in that**
the nanoparticle includes a noble metal.

4. Process according to one of the preceding claims,
**characterised in that**
the property is an optical property of the nanoparticle.

5. Process according to one of Claims 1 to 3,
**characterised in that**
in the course of the process in addition at least one colour marker is provided, and the property is an optical property of the colour marker.

6. Process according to one of the preceding claims,
**characterised in that**
the nucleic acid to be detected comprises at least two segments, and at least two nanoparticles are provided, at least one of the nanoparticles being functionalised with an oligonucleotide that is able to hybridize with the first segment of the nucleic acid, and at least one of the nanoparticles being functionalised with an oligonucleotide that is able to hybridize with the second segment of the nucleic acid.

7. Process according to one of the preceding claims,
**characterised in that**
in the case of at least some of the nanoparticles in each instance several oligonucleotides are bound to a common nanoparticle.

8. Process according to one of the preceding claims,
**characterised in that**
it runs through at least the following steps:
a) measuring the property that provides information about the degree of hybridization of the nuclei acid with the oligonucleotide at a predetermined initial temperature,
b) exciting the nanoparticle to generate heat, and
c) renewed measuring of the property that provides information about the degree of hybridization of the nucleic acid with the oligonucleotide.

9. Process according to Claim 8,
**characterised in that**
steps a) to c) are run though at least one first and one second time, the nanoparticle being excited in variably intense manner to generate heat in the course of the passes.

10. Process according to Claim 9,
**characterised in that**
in the course of each pass a melting signal is ascertained from a comparison of the property before and after the excitation of the nanoparticle, and a melting threshold is determined from the comparison of the melting signals.

11. Process according to Claim 10,
**characterised in that**
the nucleic acid is detected on the basis of a melting threshold that is specific to the nucleic acid at given initial temperature.

12. Process according to Claim 10,
**characterised in that**
the melting threshold is determined for several initial temperatures, in order to ascertain a melting-threshold curve.

13. Process according to Claim 12,
**characterised in that**
the gradient of the melting-threshold curve is ascertained.

14. Process according to Claim 12,
**characterised in that**
the melting-threshold curve is linearly extrapolated to a zero point of the melting threshold.

15. Process according to one of the following claims, **characterised in that**
the first nucleic acid is detected on the basis of an annealing temperature that is specific to the nucleic acid.

16. Process according to Claim 15,
**characterised in that**
the nucleic acid is detected by virtue of the fact that a melting threshold lies below a certain value at an initial temperature that is substantially higher than or equal to the annealing temperature.

17. Process according to Claim 15 or 16,
**characterised in that**
it runs through at least the following steps:
- ascertaining at least one melting threshold at at least one initial temperature below the annealing temperature
- temporary raising of the initial temperature to or above the annealing temperature
- ascertaining at least one melting threshold at at least one initial temperature below the annealing temperature
- comparing the melting thresholds ascertained before and after the temporary raising of the initial temperature above the annealing temperature.

18. Process according to Claim 8 or 9,
**characterised in that**
steps a) to c) are run through at least one first and one second time, the predetermined initial temperature in the course of the passes being variable, with each pass a melting signal is ascertained from a comparison of the property before and after the excitation of the nanoparticle, and the melting signals of the passes are compared.

19. Process according to one of the preceding claims,
**characterised in that**
several fractions of nanoparticles are provided which have differing excitation properties, the nanoparticles of a first fraction are functionalised for a first nucleic acid to be detected, and the nanoparticles of a second fraction are functionalised for a second nucleic acid to be detected, which is different from the first nucleic acid.

20. A kit for detecting nucleic acids, which includes:
- at least one nanoparticle that is functionalised for the nucleic acid to be detected by means of at least one oligonucleotide that is bound to it and that is able to hybridize with at least one segment of a nucleic acid to be detected,
- means for measuring a property that provides information about the degree of hybridization of the oligonucleotide with the nucleic acid to be detected,
**characterised in that**
in addition the kit includes means for optical heating of the nanoparticles.
